# EUROPEAN PATENT SPECIFICATION

(11) **EP 2 340 020 B1**
(45) Date of publication and mention of the grant of the patent: **17.12.2014**
(21) Application number: 09811182.6
(22) Date of filing: 04.09.2009
(51) Int. Cl.: C07D 257/02, A61K 31/395, C07F 13/00, C07F 15/06

(54) **MANGANESE AND COBALT COMPLEXES, THEIR PREPARATION AND USE**
MANGAN- UND KOBALTKOMPLEXE, IHRE HERSTELLUNG UND VERWENDUNG
COMPLEXES DE MANGANÈSE ET DE COBALT, LEUR SYNTHÈSE ET LEUR APPLICATION

(30) Priority: 05.09.2008 IT FI20080173
(43) Date of publication of application: 06.07.2011
(73) Proprietor: GP Investimenti S.r.l., Montespertoli (FI) (IT)
(72) Inventor: BANI, Daniele, I-50127 Firenze (IT); BENCINI, Andrea, I-50019 Sesto Fiorentino (IT); CANTORE, Miriam, I-51016 Montecatini Terme (IT); FAILLI, Paola, I-50129 Firenze (IT); GIORGI, Claudia, I-50134 Firenze (IT); VALTANCOLI, Barbara, I-50019 Sesto Fiorentino (IT)
(74) Representative: Brighenti, Livio
(86) International application number: PCT/IB2009/053859
(87) International publication number: WO 2010/026545

(56) References cited:
- WO-A-02/055544
- DE-A1- 19 816 370
- US-A1- 2005 260 259
- "Aldrich Advanced Science: Handbook of fine chemicals" 2006, ALDRICH , XP002538943 page 2509, column 2

## Description

### Field of the invention

The present invention refers to the field of use of products in combating cellular and organic damage due to oxidative stress.

### State of the art

Reactive oxygen species (ROS) form during numerous physiological processes such as mitochondrial respiration, or in the course of reactions involving enzymes that catalyze oxide-reduction reactions (lipoxygenase, cytochrome p450, NAD(P)H oxidase, nitric oxide synthetase and xanthine oxidase). There are several known species of ROS, among which the most often studied at biological level is the superoxide anion (O₂⁻). Given the high reactivity of ROS with biological molecules such as DNA, proteins and membrane lipids, their physiological levels are controlled by numerous enzymatic and other systems responsible for maintaining redox homoeostasis. These natural antioxidants include glutathione, catalase, vitamin E and superoxide dismutase (SOD), an enzyme containing manganese that represents one of the most powerful natural endogenous O₂⁻ scavengers.

An imbalance between the cellular generation of ROS, and of O₂⁻ in particular, and its inactivation by the cell is implicated in the pathogenesis of numerous inflammatory and degenerative diseases, including arteriosclerosis, rheumatoid arthritis, arthritis, bronchial asthma, reperfusion syndrome in acute myocardial infarction, cataract and periodontal disease, as well as in ageing processes. Numerous therapeutic approaches consequently focus on reducing the O₂⁻ and the damage it causes by means of exogenous molecules that take effect in association with, or in lieu of the cell's physiological control systems. Among the synthetic molecules active as O₂⁻ scavengers, some mimic the functional structure of SOD, in that they contain transition metals that are fundamental to the dismutation reaction of this radical species.

Document DE 198 16 370 A1 describes substituted Mn complexes useful as contrast agents in NMR-diagnostic.

WO 02/055544 describes fibrin-binding polypeptides, comprising a chelator, for the detection and treatment of pathological intravascular thrombosis.

Given the above considerations, the importance of making available new products capable of effectively countering the effects of free radicals is self-evident.

US2005/02600269 relates to compositions comprising a carrier with a metal binding domain, a metal ion and GLP-1, wherein 1,4,7,10-tetraazacyclododecane-N,N',N",N"'-tetraacetic acid is mentioned as the metal binding domain.

### Summary of the invention

The use of complexes of Manganese(II) or Cobalt(II) with polyamine ligands is described, that are useful as products for countering the cellular and organic damage due to oxidative stress.

### Brief description of the figures

Fig. 1 is a diagram showing the time it takes to oxidise 50% of the NBT in baseline conditions and in the presence of a compound according to the invention.
Fig. 2 is a diagram showing the percentage reduction in the O₂⁻ levels generated by macrophages activated with fMLP in the presence of various Mn(II) compounds.
Fig. 3 is a diagram showing the viability of cultures of RAW 264.7 macrophages after adding increasing concentrations of a product according to the invention.
Fig.4 shows the MDA levels measured in cells cultivated in the presence of xanthine oxidase/xanthine or fMLP-stimulated macrophages.

### Detailed description of the invention

The present invention refers to the use of complexes of Manganese(II) and Cobalt(II) with polyamine ligands with the generic formula (I):

[M(A)]·xH₂O (I)

where M = Mn or Co
x is comprised between 0 and 10
and A is a polyamine ligand with the formula (II) 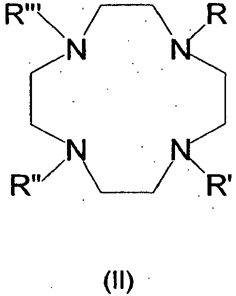
where identical or different R, R', R" and R"' groups are chosen in the group consisting of (CH₂)ₙ-COO⁻ groups, where n may be 1 or 2, linear or branch-chained C₁-C₆ alkyl groups, possibly substituted with 1 to 3 atoms of oxygen, considering that at least two of these groups are (CH₂)ₙ-COO⁻ groups where n may be 1 or 2, for the preparation of pharmaceutical compositions for treating chronic and degenerative inflammatory diseases.

In the complexes of formula (I), the metallic ion is coordinated by the four nitrogen atoms of the ligand and by the two carboxylated anionic groups, and it can consequently be represented, for instance, with the two structures shown below (where the substituents are as defined above) according to whether the acetate groups are respectively in position 1,4 or 1,7 in relation to one another.

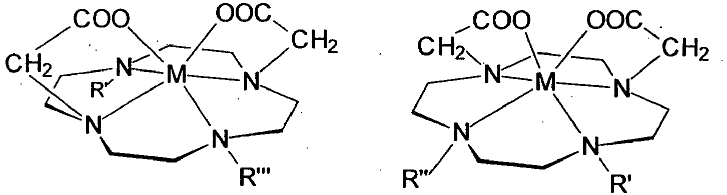

By alkyl groups according to the invention, we mean in particular: methyl, ethyl, propyl, iso-propyl, butyl, t-butyl, pentyl, 2-methyl-butyl, dimethyl-propyl, hexyl, 2,2-dimethyl-butyl, 2,3-dimethyl-butyl, 2-methyl-pentyl, 3-methyl-pentyl, cyclohexyl. Preferred alkyl groups are methyl, ethyl, propyl and isopropyl.

The product used for the coordination of the metal according to the invention can be prepared according to essentially known methods (see for instance P. barbaro et al. J. Chem. Soc., Dalton Trans., 2000, 2393); normally the ligand is prepared and then made to react with the required medal, generally in the form of a watersoluble salt.

For instance, to prepare the ligands with the two acetate groups in positions 1 and 7, an aqueous solution of chloroacetic acid is added to an aqueous solution of alkylated 1,4,7,10-tetrazacyclododecane in positions 4 and 10 (for an example of the derivative synthesis of 1,4,7,10-tetrazacyclododecane dialkylate in positions 4 and 10, see M. Ciampolini et al., J. Chem. Soc., Dalton Trans., 1984, 1357), adjusting the pH to 9-10 with the addition of an alkaline aqueous solution. The resulting solution is heated gently to 50-50°C for 16-24 hours, maintaining a constant pH with further additions of the alkaline solution.

The solution is then reduced to dryness in the rotary evaporator, restored with water and brought to a neutral pH with an acid solution.

The solution is submitted to chromatography in a column containing a resin such as Amberlite in acid form.

The product is diluted first with water, then with a solution of ammonia, and finally with water once again. The fractions obtained are reduced to dryness and analysed by ¹H NMR.

The fractions containing the product required are pooled together, dissolved in water and the solution is chromatographed on resin such as Amberlite in alkaline form.

The product is diluted first with water, then with a solution of more concentrated HCl, and finally with a solution of more diluted HCl. The fractions are individually reduced to dryness and analysed by ¹H NMR. Those containing the product desired were suspended in ether and filtered, finally obtaining the product desired in the form of a hydrochloride salt.

The ligand thus obtained was dissolved in water and an alkaline solution was added to neutralise the hydrochloric acid. The solution was deoxygenated by heating and flushing with nitrogen bubbles. A previously deoxygenated solution of a soluble Manganese(II) or Cobalt(II) salt, preferably a chloride, was slowly added drop by drop to the solution after heating it to boiling point, then it was kept boiling for approximately half an hour, subsequently evaporating the solvent to near dryness in a flow of nitrogen. When the solution returned to ambient temperature, ethyl alcohol and diethyl ether were added to obtain a precipitate that was filtered under nitrogen and washed with EtOH.

The solid obtained can be purified to remove any NaCl by means of crystallisation with ethanol or ethanol/water or by nanofiltration.

The invention is better illustrated and explained in the light of the following examples.

### Example 1

Synthesis of the 4,10-dimethyl-1,4,7,10-tetraazacyclododecane-1,7-diacetic acid trihydrochloride ligand

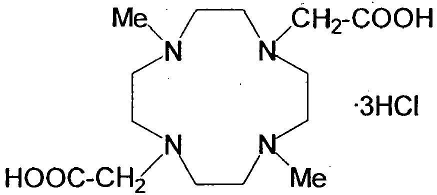

A solution of chloroacetic acid (5.98 g, 63 mmol) in 10 ml of water was added to a solution of 1,10-dimethyl-1,4,7,10-tetraazacyclododecane (for the synthesis of this compound, see M. Ciampolini et al., J. Chem. Soc., Dalton Trans., 1984, 1357) (1.26 g, 6.3 mmol) in 10 ml of water. The pH of the resulting solution was adjusted to 9.5 with an aqueous solution of NaOH 5 M. The solution was kept at 70°C for 18 h, maintaining the pH at 9.5 by means of small additions of NaOH 5 M. The solution was then reduced to dryness in the rotary evaporator, restored with 20 ml of water and adjusted to a neutral pH with HCl 5M. The solution was deposited in a column containing Amberlite IR 120 (volume 60 cm³) in acid form (the resin is activated by a treatment of the original resin [Na⁺ form] first with 500 HCl 0.1 M and then with 400 ml of water). The product was diluted first with 500 ml of water, then with a solution of ammonia 0.5 M (600 ml) and finally with 700 ml of water Fractions of approximately 100 ml were collected and dried separately and analysed by ¹H NMR. The fractions containing the product required were combined, dissolved in 10 ml of water and the solution was deposited in a column Amberlite IRA 900 (volume of the bed 50 cm³) in alkaline form (the activation of the rating is achieved by means of treatment of the original resin [forma Cl⁻] first with 400 ml of NaOH 0.1 M and subsequently with 350 ml of water). The product was diluted first with 350 ml of water, then with a solution of HCl 5 M (300 ml) and finally with 300 ml of molar HCl 0.1. The fractions (approximately 50 ml each) were separately reduced to dryness and analysed by ¹H NMR. Those containing the required product were suspended in ether and filtered to obtain a white product as required (obtaining 1.35 g, 50.7%).
Elementary analysis calculated for C₁₄H₂₈N₄O₄·3HCl (%): C, 39.49; H, 7.34; N, 13.16; Cl 24.98
We found: C, 39.59; H, 7.54; N, 13.14; Cl 25.10.
¹H NMR (300.07 MHz, D₂O, pH 3.0, 25°C), δ(ppm): 3.57 (4H, s); 3.49 (4H, dd); 3.31 (4H, dd); δ 3.17 (4H, dd); δ 2.16 ppm (4H, dd); ¹³C NMR (50.3 MHz, D₂O, pH 3.0, 25°C), δ(ppm): 177.0; 54.9; 54.3; 50.1.
ESI mass spectrum: 317 (H₂L + H⁺)

### Example 2

### Synthesis of the complex Mn(4,10-dimethyl-1,4,7,10-tetraazacyclododecane-1,7-diacetate) 2H₂O)

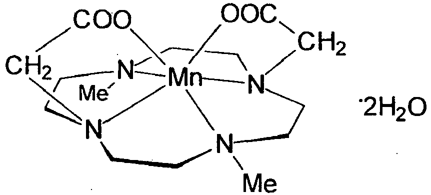

100 mg of the ligand obtained as in Example 1 (0.23 mmol) were dissolved in 10 ml of water, then 0.69 ml of a 1 M solution of NaOH (0.69 mmol) were added to the resulting solution. The solution was deoxygenated by flushing N₂ bubbles for approximately 30 min through the solution heated to a 80°C. The solution was heated to boiling point, then a previously deoxygenated solution of MnCl₂·4H₂O (42 mg, 0.21 mmol) in 10 ml of water was added drop by drop over approximately 30 minutes. After completing this addition, the solution was kept boiling for 30 minutes and then evaporated to 5 ml in a flow of nitrogen. The solution was restored to ambient temperature, then 20 ml of EtOH and 10 ml of diethyl ether were added, obtaining a white precipitate that was filtered under nitrogen and washed with EtOH.

To eliminate any NaCl, the solid obtained was dissolved in a little water (approximately 3 ml) and EtOH (approximately 50 ml) was added to the solution obtained. The suspension was concentrated to approximately 30 ml and then filtered. The required complex was recovered in a subsequent further concentration of the solution to approximately half its volume, adding EtOH (20 ml) and cooling to 4°C overnight, thus obtaining 45 mg (52.9 %).
Elementary analysis calculated for C₁₄H₃₀N₄O₆Mn (%): C, 41.48; H, 7.46; N, 13.82; Mn 13.55.
We found C, 41.28; H, 7.26; N, 13.66; Mn 13.34.
ESI mass spectrum: 370 (Mn-ligand + H⁺).

The compounds of formula (I) can be administered in the form of:
1 - isotonic solutions (e.g. NaCl 0.9%, phosphate buffer 0.1 M, pH 7.4, Ringer lactate buffer, etc.), sterilised by filtration, for parenteral and topical uses (intravenous, percutaneous or intra-articular uses, local infiltrations, eye drops, mouthwashes);
2 - gels and creams (products containing standard excipients in the current pharmacopoeia) for topical, cutaneous and mucosal applications;
3 - toothpastes (products containing standard excipients in the current pharmacopoeia) for topical applications in dentistry.

These formulations are prepared using standard pharmacological techniques and the quantities of active ingredient contained in the preparations normally is comprised between 0.5 and 50 mg/ml.

Given its chemical properties, the compound described herein has proved capable of effectively reducing the levels of superoxide anion (O₂⁻) that, as explained previously, is known to play a key part in inflammatory damage in cells and tissues.

In particular, the results of experiments conducted at the laboratories of the inventors at the Faculty of Medicine of the University of Florence demonstrated that the compound reduces the measurable levels of O₂⁻, generated either by means of an enzymatic system *in vitro* or by stimulating inflammatory cells in cultures. Moreover, the compound distinctly attenuated the harmful effects of O₂⁻ on the cells, consequently proving effective in reducing oxidative stress, which plays a fundamental part in the pathogenesis of numerous chronic and degenerative inflammatory diseases. These effects can be documented at very low concentrations of the compound, of the order of 0.1 milligrams/litre, which goes to show its high biological efficacy; at these concentrations it has revealed no cell toxicity.

### Example 3

### O²⁻ scavenging in a cell-free system (xanthine/xanthine oxidase)

*In vitro,* the xanthine oxidase enzyme metabolises the xanthine substrate and generates uric acid and O₂ as products of the reaction, which can be measured spectrophotometrically by oxidation of the NBT chromogen.

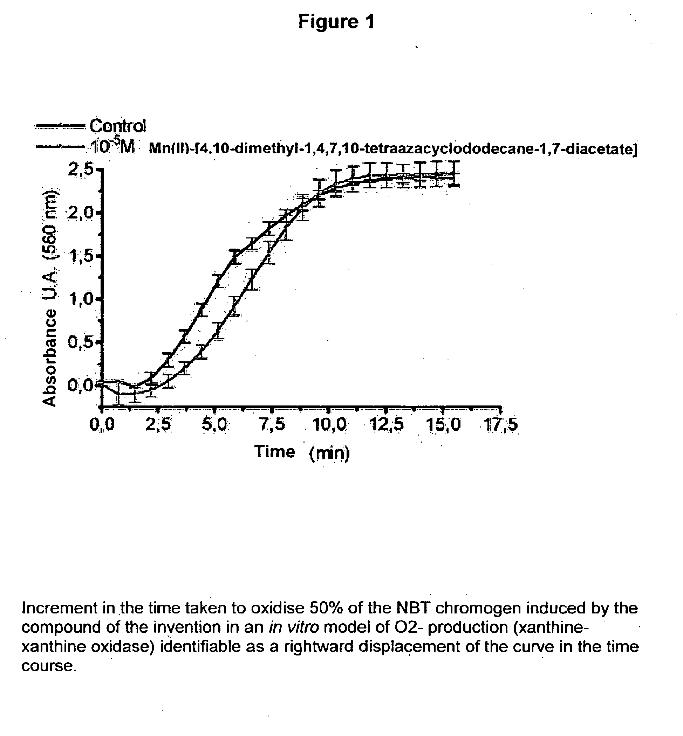

In this system, the compound of Example 2 (10⁻⁵ M) determines a significant increase in the time taken to oxidise 50% of the NBT (294±12 sec. in baseline conditions; 390±16 sec. in the presence of the compound; *p*<0.01), which demonstrates that this compound reduces the levels of O₂⁻ in the reaction buffer (see Figure 1).

This effect appears to be due to a specific scavenging action on the O₂⁻, not to any generic inhibition of the xanthine oxidase enzyme, since the compound does not reduce the levels of uric acid.

Other Mn(II) compounds with different organic scaffolds, tested in the same system, had no or only a markedly more limited efficacy.

### Example 4

### O₂⁻ scavenging in cultures of fMLP-stimulated RAW 264.7 macrophages

The chemotactic peptide fMLP triggers a specific inflammatory activation of the macrophages, which produce high levels of O₂⁻, that are measurable spectrophotometrically by means of the oxidation of the Fe(II)cytC chromogen.

In this system, the compound of Example 2 (10⁻⁹-10⁻⁶ M) determines a dose-dependent, significant reduction in the levels of oxidised Fe(II)cytC, which goes to show that this compound reduces the levels of O₂⁻ endogenously generated by the macrophages activated and released in the culture medium.

The 50% inhibiting concentration (IC50%) amounts to 6x10⁻⁸ M.

The compound remains active even when pre-incubated and then removed from the cell cultures before adding the activating stimulus, which demonstrates that its partial lipophilia enables it to penetrate inside the cell and exert its scavenging function at intracellular level.

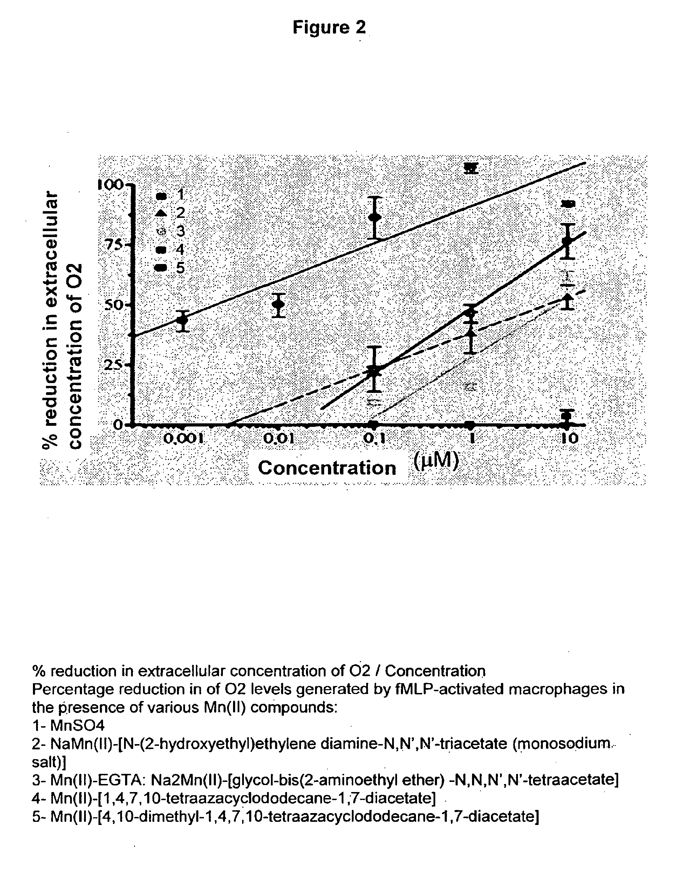

Other Mn(II) compounds with different organic scaffolds, tested in the same system, were markedly less effective. MnSO₄, used as a negative control, was entirely ineffective (see Figure 2).

### Example 5

### Toxicity of Mn(II)Me₂DO2A in cultures of RAW 264.7 macrophages

The viability of the cultures of RAW 264.7 macrophages was measured at various intervals (24, 48, 72 h) after adding increasing concentrations of the compound of Example 2 (10⁻⁷ M- 10⁻⁴ M).

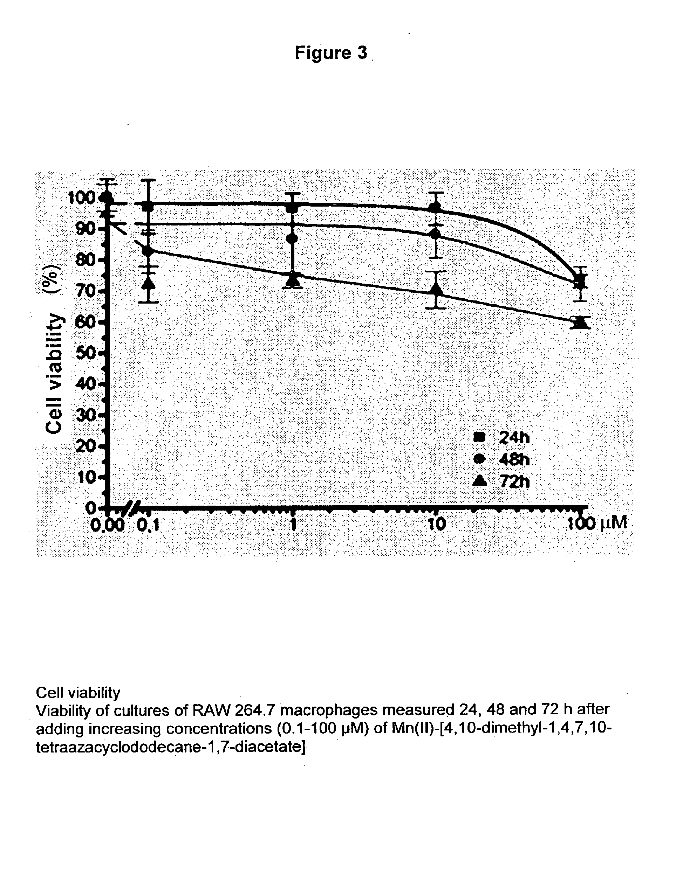

In this system, the compound described herein demonstrated an excellent therapeutic profile, since it proved mildly cytotoxic only after incubation times of 72 hours and at the greatest concentrations (10⁻⁵-10⁻⁴ M), far higher than those at which the compound was active as a scavenger (see Figure 3).

### Example 6

### Protective effect of the compound of Example 2 on cellular damage induced by oxidative stress

The primary targets of the cytotoxic action of ROS are the membrane lipids, which are peroxidised with the formation of malondialdehyde (MDA). This can be measured spectrophotometrically using the chromogen that is generated from the reaction of MDA with thiobarbituric acid. This measurement is consequently a reliable indicator of ROS-induced cell damage.

Cultures of rat aorta smooth muscle cells (RASM) were prepared in a medium in which O₂⁻ was generated both by means of the xanthine oxidase/xanthine enzymatic system and by RAW 264.7 macrophages stimulation with fMLP (see the two diagrams shown below, respectively).

In both systems, the compound tested significantly reduced the levels of MDA in the RASM cells.

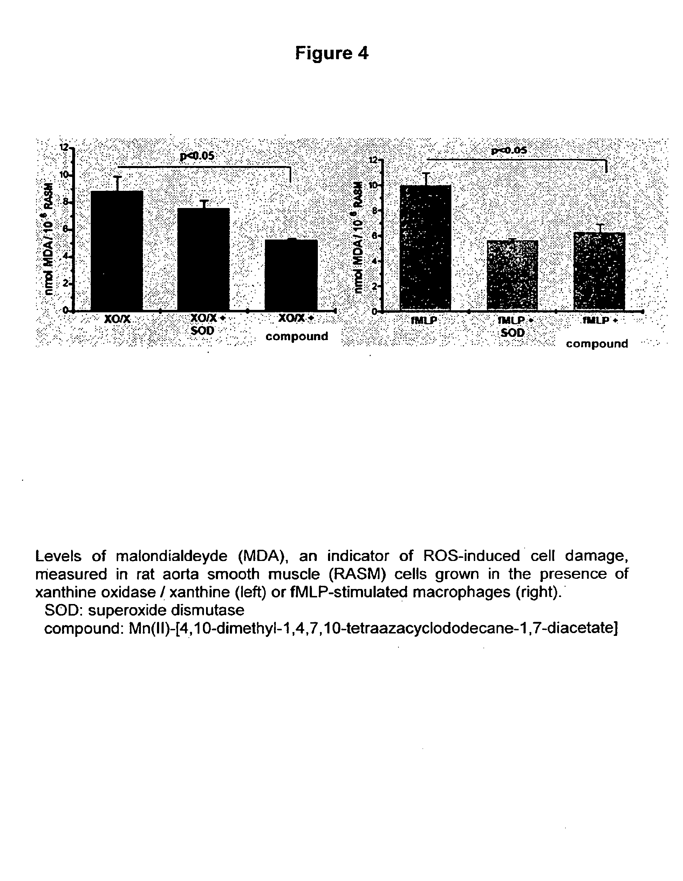

These effects were comparable with, or even greater than those achievable using the enzyme superoxide dismutase (SOD), which is a well-known inactivator of O₂⁻ (see Figure 4).

Overall, the above *in vitro* data indicate that the compound of Example 2 is a powerful and specific O₂⁻ scavenger capable of reducing the cellular damage due to oxidative stress, with virtually no cytotoxicity at effective concentrations. Thanks to its partial lipophilia, the compound becomes distributed in the intracellular volume, where it maintains its properties.

By comparison with SOD (the enzyme currently used to reduce O₂⁻ levels in the extracellular environment), the compound described herein represents a substantial improvement in that it is at least as pharmacologically effective at intracellular level too.

## Claims

1. Use of complexes of Manganese(II) or Cobalt(II) with polyamine ligands having the generic formula (I):
[M(A)] ▪ xH₂O (I)
where M is chosen between Mn (II) and Co (II);
x is comprised between: 0 and 10
and A is a polyamine ligand of formula (II) 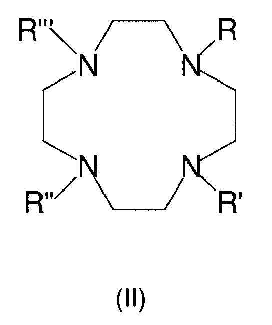
where identical or different R, R', R" and R'" groups are chosen in the group consisting of (CH₂)ₙ-COO⁻ groups, where n is selected from 1 or 2, linear or branch-chained C₁-C₆ alkyl groups, possibly substituted with 1 to 3 atoms of oxygen, considering that at least two of these groups are (CH₂)ₙ-COO⁻ groups where n is as defined above, and where the metallic ion is coordinated by the four nitrogen atoms of the ligand and by the two carboxylated anionic groups for the preparation of pharmaceutical compositions for treating chronic and degenerative inflammatory diseases.

2. Use according to claim 1, wherein the alkyl is chosen from among: methyl, ethyl, propyl, iso-propyl, butyl, t-butyl, pentyl, 2-methyl-butyl, dimethyl-propyl, hexyl, 2,2-dimethyl-butyl, 2,3-dimethyl-butyl, 2-methyl-pentyl, 3-methyl-pentyl, cyclohexyl.

3. Pharmaceutical compositions for treating chronic and degenerative inflammatory diseases containing a compound of formula (I) according to claims 1 - 2 as the active ingredient, and any excipients.

4. Pharmaceutical compositions for use according to claim 3, wherein said chronic and degenerative inflammatory diseases are chosen among: arteriosclerosis, rheumatoid arthritis, arthritis, bronchial asthma, reperfusion syndrome in acute myocardial infarction, cataract, periodontal diseases.

5. Compositions for use according to claims 3 and 4, in the form of:
- isotonic solutions sterilised by filtering;
- gels and creams for topical, cutaneous or mucosal applications;
- toothpastes for topical applications in dentistry.

## Patentansprüche

1. Verwendung von Komplexen von Mangan (II) oder Cobalt (II) mit Polyaminliganden mit der allgemeinen Formel (I):
[M(A)] · xH₂O (I),
worin M aus Mn (II) und Co (II) ausgewählt wird,
x 0 bis 10 beträgt und
A ein Polyaminligand gemäß der Formel (II) ist: 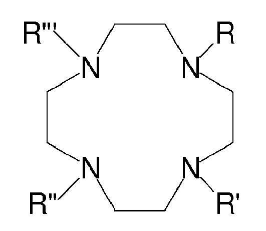
worin R, R', R" und R'" gleiche oder verschiedene Gruppen ausgewählt aus der Gruppe bestehend aus (CH₂)ₙ-COO⁻-Gruppen, worin n 1 oder 2 ist, linearen oder verzweigtkettigen C₁-C₆-Alkylgruppen, gegebenenfalls substituiert mit 1 bis 3 Sauerstoffatomen sind mit der Maßgabe, dass wenigstens zwei dieser Gruppen (CH₂)ₙ-COOO⁻-Gruppen sind, wobei n wie zuvor definiert ist, und, wobei das Metallion mit den vier Stickstoffatomen des Liganden und mit den zwei carboxylierten anionischen Gruppen koordiniert ist, zur Herstellung von pharmazeutischen Zusammensetzungen zur Behandlung von chronischen und degenerativen Entzündungserkrankungen.

2. Verwendung nach Anspruch 1, wobei die Alkylgruppe aus Methyl, Ethyl, Propyl, Iso-Propyl, Butyl, t-Butyl, Pentyl, 2-Methyl-butyl, Dimethyl-propyl, Hexyl, 2,2-Dimethyl-butyl, 2,3-Dimethyl-butyl, 2-Methyl-pentyl, 3-Methyl-pentyl, Cyclohexyl ausgewählt wird.

3. Pharmazeutische Zusammensetzungen zur Behandlung von chronischen und degenerativen Entzündungserkrankungen, welche eine Verbindung gemäß der Formel (I) nach Anspruch 1 oder 2 als aktivem Bestandteil und irgendwelche Hilfsmittel enthalten.

4. Pharmazeutische Zusammensetzungen zur Verwendung nach Anspruch 3, wobei die chronischen und degenerativen Entzündungserkrankungen aus Arteriosklerose, rheumatoider Arthritis, Arthritis, Bronchialasthma, Reperfusionssyndrom in akuten Myokardinfarkten, Katarakt, periodontaler Erkrankungen ausgewählt werden.

5. Zusammensetzungen zur Verwendung nach Anspruch 3 und 4 in der Form von:
- isotonischen Lösungen, welche durch Filtrieren sterilisiert worden sind,
- Gelen und Cremes für topische, kutane oder mukosale Anwendungen,
- Zahnpasten für topische Anwendungen in der Zahnmedizin.

## Revendications

1. Utilisation de complexes de manganèse (II) ou de cobalt (II) avec des ligands polyaminés ayant la formule générique (I) :
[M(A) ] . xH₂O (I)
où M est choisi entre Mn (II) et Co (II) ;
x est compris entre : 0 et 10
et A est un ligand polyaminé de formule (II) 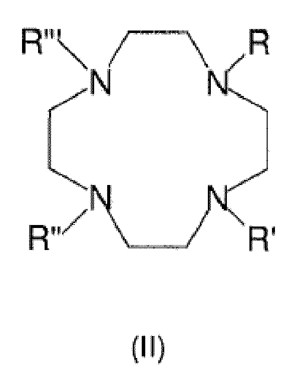
où les groupes R, R', R'' et R"', identiques ou différents, sont choisis dans le groupe constitué des groupes (CH₂)ₙ-COO⁻, où n est choisi parmi 1 ou 2, les groupes alkyle en C₁ à C₆ à chaîne linéaire ou ramifiée, éventuellement substitués avec 1 à 3 atomes d'oxygène, en considérant qu'au moins deux de ces groupes sont des groupes (CH₂)ₙ-COO⁻ où n est tel que défini ci-dessus, et où l'ion métallique est coordonné par les quatre atomes d'azote du ligand et par les deux groupes anioniques carboxylés
pour la préparation de compositions pharmaceutiques destinées au traitement des maladies inflammatoires chroniques et dégénératives.

2. Utilisation selon la revendication 1, dans laquelle l'alkyle est choisi parmi : le méthyle, l'éthyle, le propyle, l'isopropyle, le butyle, le t-butyle, le pentyle, le 2-méthyl-butyle, le diméthyl-propyle, l'hexyle, le 2,2-diméthyl-butyle, le 2,3-diméthyl-butyle, le 2-méthyl-pentyle, le 3-méthyl-pentyle, le cyclohexyle.

3. Compositions pharmaceutiques destinées au traitement des maladies inflammatoires chroniques et dégénératives, contenant un composé de formule (I) selon les revendications 1 à 2 en tant que principe actif, et des excipients.

4. Compositions pharmaceutiques pour leur utilisation selon la revendication 3, dans lesquelles lesdites maladies inflammatoires chroniques et dégénératives sont choisies parmi : l'artériosclérose, la polyarthrite rhumatoïde, l'arthrite, l'asthme bronchique, le syndrome de reperfusion dans le cas d'un infarctus aigu du myocarde, la cataracte, les parodontopathies.

5. Compositions pour leur utilisation selon les revendications 3 et 4, sous la forme de :
- solutions isotoniques stérilisées par filtration ;
- gels et crèmes pour des applications topiques, cutanées ou sur les muqueuses ;
- pâtes dentifrices pour des applications topiques en dentisterie.
